# EUROPÄISCHE PATENTSCHRIFT

(11) **EP 4 035 719 B1**
(45) Veröffentlichungstag und Bekanntmachung des Hinweises auf die Patenterteilung: **13.08.2025**
(21) Anmeldenummer: 22155423.1
(22) Anmeldetag: 14.09.2017
(51) Int. Cl.: A61M 16/20, B01L 3/00, F16K 99/00, A61M 11/00, F04B 19/00, A61M 39/24

(54) **VORRICHTUNG ZUR VERABREICHUNG EINES FLÜSSIGEN MEDIKAMENTS**
DEVICE FOR ADMINISTERING A LIQUID MEDICAMENT
DISPOSITIF D'ADMINISTRATION D'UN MÉDICAMENT LIQUIDE

(30) Priorität: 15.09.2016 DE 102016117396
(43) Veröffentlichungstag der Anmeldung: 03.08.2022
(62) Teilanmeldung aus: 17778198.6
(73) Patentinhaber: invoX Belgium NV, 3590 Diepenbeek (BE)
(72) Erfinder: BARTELS, Frank, 45527 Hattingen (DE); RAWERT, Jürgen, 50933 Köln (DE)
(74) Vertreter: Synergy IP Group AG

(56) Entgegenhaltungen:
- EP-A1- 2 896 457
- EP-A2- 0 153 110
- WO-A1-2005/000476
- WO-A1-2012/007315
- WO-A1-2012/098140
- WO-A1-2013/029159
- WO-A2-97/24528
- DE-A1- 10 330 370
- DE-A1- 4 236 037
- US-A1- 2004 159 319
- US-A1- 2007 160 474
- US-A1- 2012 138 713

## Beschreibung

### Hintergrund der Erfindung

Die Erfindung betrifft eine Vorrichtung zur Verabreichung eines flüssigen Medikaments.

### Beschreibung

Aus dem Stand der Technik sind Vorrichtungen zur Verabreichung eines flüssigen Medikaments bekannt, welche ein Reservoir aufweisen, das mit einem Pumpensystem verbunden ist. Die Auslassseite der Pumpe ist mit einem Medikamentenauslass verbunden, beispielsweise mit einem Rohr oder Schlauch, oder mit einem Zerstäuber. Häufig weist die Pumpenkammer der Pumpe ein Einlass- und ein Auslassventil auf. Das Einlassventil verschließt die Pumpenkammer genau dann, wenn die Pumpe einen Ausgabedruck zur Bereitstellung des Medikaments über das Rohr, den Schlauch, oder den Zerstäuber erzeugt, um ein Zurückfließen des Medikaments in das Reservoir zu vermeiden. Für das Wiederauffüllen der Pumpenkammer wird in der Pumpenkammer ein Unterdruck erzeugt, wodurch das Medikament aus dem Reservoir durch das sich öffnende Einlassventil in die Pumpenkammer fließt, während das Auslassventil schließt, um ein Zurückfließen des Medikaments aus dem Rohr, Schlauch, oder Zerstäuber zu vermeiden. Die Ventile sind somit als gängige Einwegventile, beispielsweise als Rückschlagventile, ausgebildet. Ähnliche Vorrichtungen beschreiben die WO 2013/191011 A1, die US 8,628,517 B2 und die WO 2013/072790 Al.

WO 2012/007315 offenbart ein Gerät zum Verabreichen einer flüssigen medizinischen Formulierung, die sich in einem in das Gerät eingesetzten Behälter befindet und durch mindestens eine Düsenöffnung aus dem Gerät ausgebracht wird, wobei die flüssige medizinische Formulierung vor Durchströmen der mindestens einen Düsenöffnung durch einen Feinstfilter strömt, der von einem mikro strukturierten Bauteil gebildet wird.

Insbesondere bei medizinischen Anwendungen ist es häufig wünschenswert, dass die Vorrichtung zur Verabreichung des flüssigen Medikaments möglichst klein baut und somit wenig Platz einnimmt. Insbesondere die aus dem Stand der Technik bekannten, üblicherweise rein mechanischen Einlass- und Auslassventile können jedoch nicht unbeschränkt verkleinert werden, so dass Bedarf danach besteht, derartige Ventile noch weiter zu verbessern, oder sie gegebenenfalls sogar ganz überflüssig zu machen.

Es ist daher die Aufgabe der Erfindung, ein gattungsgemäßes Ventil und eine entsprechende Vorrichtung zur Verabreichung eines flüssigen Medikaments vorzuschlagen, welche möglichst geringe Abmessungen aufweisen.

Diese Aufgabe wird erfindungsgemäß durch eine Vorrichtung mit den Merkmalen des Patentanspruchs 1 gelöst. Die abhängigen Ansprüche 2 bis 14 betreffen jeweils vorteilhafte Ausführungsformen der Erfindung.

Die Erfindung betrifft eine Vorrichtung zur Verabreichung eines flüssigen Medikaments, mit einem Reservoir, in dem ein Medikament vorgehalten oder vorhaltbar ist, und mit einer Pumpe, die eine Pumpenkammer aufweist, die über ein nur in Richtung vom Reservoir in die Pumpenkammer durchlässiges Einwegventil mit dem Reservoir fluidisch verbunden ist, und die über ein erfindungsgemäßes Ventil mit einem Mcdikamentenauslass fluidisch verbunden ist.

Das erfindungsgemäße Ventil weist einen Ventilkörper auf, der einen Innenraum zur Aufnahme einer Flüssigkeit, insbesondere eines flüssigen Medikaments, umfasst. Der Ventilkörper weist einen Flüssigkeitseinlass und einen gegenüberliegenden Flüssigkeitsauslass auf, die beide in den Innenraum münden. In dem Innenraum ist eine Vielzahl Mikrokanäle angeordnet, die sich in Verbindungsrichtung zwischen dem Flüssigkeitseinlass und dem Flüssigkeitsauslass erstrecken.

Das erfindungsgemäße Ventil macht sich den Kapillareffekt zunutze. Es ist bekannt, dass Flüssigkeiten aufgrund von Kapillarkräften Oberflächen benetzen und durch komplexe Strukturen hindurchbewegen können. Die für die Flüssigkeitsbewegung erforderliche Energie wird dabei durch den Unterschied der atomaren Anziehungskräfte zwischen den Flüssigkeitsatomen im Flüssigkeitsinneren und den atomaren Anziehungskräften zwischen den Flüssigkeitsatomen, welche sich an der Flüssigkeitsoberfläche und damit an der Grenzfläche zwischen der Flüssigkeit und einem Gas befinden. Die Grenzfläche zwischen einer Flüssigkeit und einem Gas wird auch als freie Oberfläche bezeichnet. Ebenso muss für die Entfernung von Flüssigkeitsatomen aus der freien Oberfläche Energie aufgewendet werden, so dass vormals tiefer liegende, sich im Flüssigkeitsinneren befindliche Atome die freie Oberfläche bilden. Es muss daher eine Kraft aufgewendet werden, um Flüssigkeitsanteile von stark benetzenden Oberflächen zu entfernen.

Der Ventilkörper bildet einen Flüssigkeitskanal, der von Seitenwänden begrenzt ist. Die Seitenwände können parallele Seitenwände eines im Querschnitt polygonalen oderrunden, beispielsweise kreisrunden Kanals sein.

Der Innenraum weist eine Querschnittsfläche auf, die größer als eine Querschnittsfläche der Mikrokanäle ist, wobei ein Querschnittsflächenverhältnis zwischen den Mikrokanälen und dem Innenraum vorzugsweise zwischen 1:5 und 1:1000 und besonders bevorzugt zwischen 1:50 und 1:100 beträgt. Die Mikrokanäle weisen vorzugsweise einen Durchmesser zwischen 1 µm und 200 µm und besonders bevorzugt zwischen 5 µm und 20 µm auf.

Erfindungsgemäß sind die Mikrokanäle von einem Gitter aus parallelen, stabförmigen Begrenzungselementen, oder von mehreren parallelen, zueinander versetzt angeordneten Lagen eines Gitters aus parallelen, stabförmigen Begrenzungselementen gebildet. Die Begrenzungselemente können sich dabei senkrecht zur Verbindungsrichtung zwischen dem Flüssigkeitseinlass und dem Flüssigkeitsauslass erstrecken. Die Begrenzungselemente weisen vorzugsweise eine runden, insbesondere kreisrunden, oder einen polygonalen Querschnitt auf. Die Begrenzungselemente können beispielsweise einen Durchmesser zwischen 0,5 µm und 50 µm, bevorzugt zwischen 3 µm und 15 µm aufweisen. Die Länge der Begrenzungselemente kann anbei von einigen µm bis zum vollen Durchmesser des Innenraums des Ventilkörpers betragen, besonders bevorzugt beträgt die Länge der Begrenzungselemente zwischen 20% und 80% des Innenraumdurchmessers senkrecht zur Verbindungsrichtung zwischen Flüssigkeitseinlass und Flüssigkeitsauslass. Dazu können sich die Begrenzungselemente ausgehend von einer Innenseite der den Innenraum des Ventilkörpers begrenzenden Seitenwand in Richtung einer gegenüberliegenden Seitenwand erstrecken, ohne diese zu erreichen, so dass ein Abstand zwischen den Begrenzungselementen und der jeweils gegenüberliegenden Seitenwand ausgebildet ist. Diese Ausführungsform zeichnet sich insbesondere auch dadurch aus, dass sie einfach in der Herstellung ist.

Zur Erhöhung der Adhäsion zwischen den Begrenzungselementen und einer Flüssigkeit ist bei einer Ausführungsform der Erfindung vorgesehen, dass die Oberfläche der Begrenzungselemente eine funktionale Beschichtung, beispielsweise eine hydrophile Beschichtung, aufweist. Dazu kann weiterhin die Kanalinnenseite hydrophob beschichtet sein.

Erfindungsgemäß weisen der Innenraum, der Flüssigkeitseinlass und der Flüssigkeitsauslass dieselbe Querschnittsfläche senkrecht zur Verbindungsrichtung auf. Dadurch wird eine besonders kompakte und einfach herstellbare Ventilgeometrie erreicht.

Dabei kann vorgesehen sein, dass der Ventilkörper parallele Seitenwände aufweist, deren Innenseiten den Innenraum abgrenzen, wobei die Seitenwände an gegenüberliegenden Enden in den Flüssigkeitseinlass bzw. den Flüssigkeitsauslass münden. Ein einfach herstellbares Ventil wird dadurch erreicht, dass bei der zuletzt genannten Ausführungsform der Ventilkörper über seine gesamte Länge zwischen dem Flüssigkeitseinlass und dem Flüssigkeitsauslass einen konstanten Querschnitt aufweist.

Der Ventilkörper kann einen runden, insbesondere kreisrunden, oder einen polygonalen Querschnitt aufweisen.

### Kurze Beschreibung der Figuren

Weitere Einzelheiten der Erfindung werden anhand der nachstehenden Figuren erläutert. Dabei zeigt:
- Figur 1: einen schematischen Längsquerschnitt durch eine Ausführungsform des erfindungsgemäßen Ventils ohne angelegten Unterdruck;
- Figur 2: einen schematischen Längsquerschnitt des Ventils gemäß Figur 1 mit angelegtem Unterdruck P1 > 0;und
- Figur 3: einen schematischen Längsquerschnitt des Ventils gemäß den Figuren 1 und 2 mit angelegtem Unterdruck P2 > P1•

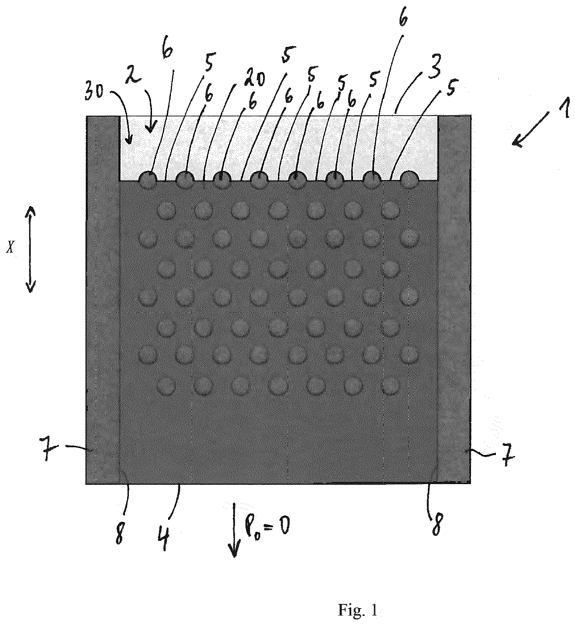

Bei dem in Figur 1 dargestellten Ventil ist der Ventilkörper 1 im Längsquerschnitt dargestellt. Der Ventilkörper 1 ist durch gegenüberliegende parallele Seitenwände 7 begrenzt. Die Seitenwände 7 begrenzen mit ihren Innenseiten 8 einen Innenraum 2, in dem eine Flüssigkeit 20, beispielsweise ein Medikament, aufgenommen ist. An gegenüberliegenden Seiten des Ventilkörpers 1 ist ein Flüssigkeitseinlass 3 bzw. ein Flüssigkeitsauslass 4 ausgebildet. Der Flüssigkeitseinlass 3 und der gegenüberliegende Flüssigkeitsauslass 4 weisen gerade denselben Querschnitt wie der übrige Ventilkörper 1, insbesondere wie der Innenraum 2 auf. An den Flüssigkeitsauslass 4 kann beispielsweise eine Pumpe mit einer Pumpenkammer einer Vorrichtung zur Verabreichung eines flüssigen Medikaments angeschlossen sein.

Der Ventilkörper 1 kann beispielsweise einen kreisrunden Querschnitt aufweisen, oder einen polygonalen, beispielsweise einen rechteckigen und insbesondere einen quadratischen. Die in Figur 1 im Querschnitt gezeigten Begrenzungselemente 6 sind sich parallel zueinander erstreckende, stabförmige Gitterstreben, welche sich senkrecht zur Zeichnungsebene erstrecken. Jeweils zwei benachbarte Begrenzungselemente 6 bilden zwischen sich einen Mikrokanal 5 aus, der sich in Verbindungsrichtung x zwischen dem Flüssigkeitseinlass 3 und dem Flüssigkeitsauslass 4 erstreckt und zu beiden Einlässen 3, 4 hin geöffnet ist.

Wie in Figur 1 dargestellt ist, sind die Mikrokanäle von mehreren parallelen, zueinander versetzt angeordneten Lagen eines Gitters aus parallelen, stabförmigen Begrenzungselementen gebildet.

Wenn an dem Flüssigkeitsauslass 4 kein Unterdruck anliegt (Po= 0), bildet die Flüssigkeit 20, wie in Figur 1 gezeigt ist, eine im Wesentlichen ebene freie Oberfläche zwischen sich und dem Gas 30aus.

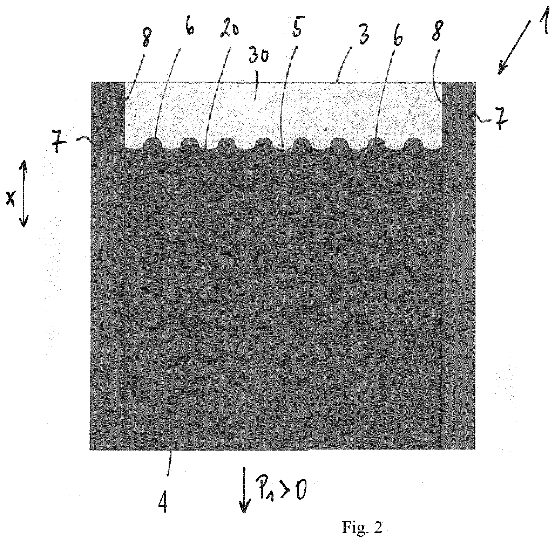

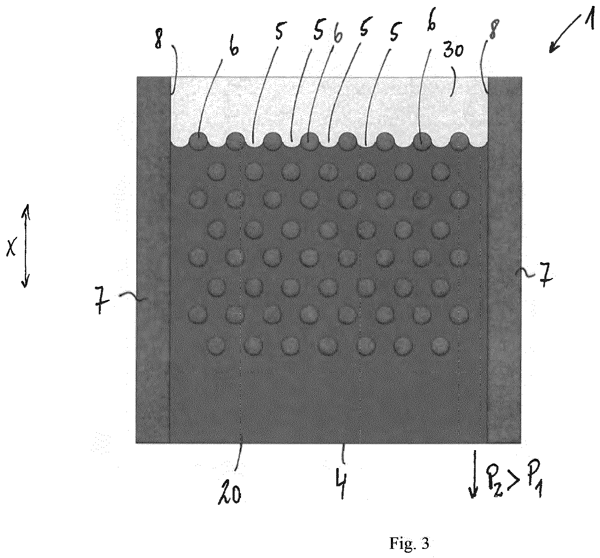

Erst wem1 ein Unterdruck (P1 > 0) an dem Flüssigkeitsauslass 4 anliegt (siehe Figur 2), bildet die freie Oberfläche zwischen der Flüssigkeit 20 und dem Gas 30 eine konkave Geometrie aus. Mit wachsendem Unterdruck sinkt der Radius der konkaven Grenzfläche zwischen Flüssigkeit 20 und Gas 30. In Figur 3 ist der Fall gezeigt, bei dem P2 > P1 gilt.

Der Krümmungsradius der freien Oberfläche ist abhängig vom sogenannten Laplace-Druck. Dieser Druck steigt mit kleiner werdendem Grenzflächenradius. Wenn daher der Unterdruck den für die in den Figuren 1 bis 3 dargestellte Mikrokanalstruktur gültigen maximalen Laplace-Druck übersteigt, wird die Flüssigkeit aus dem Ventilkörper 1 heraustransportiert. Das erfindungsgemäße Ventil eignet sich daher insbesondere für die Verwendung als Auslassventil bei einer gattungsgemäßen Vorrichtung zur Verabreichung eines flüssigen Medikaments.

Grundsätzlich steigt der Laplace-Druck proportional zur Oberflächenspannung der Flüssigkeit. Um daher bei gegebener Oberflächenspannung den Schwellwert für den Unterdruck, bei dem die Flüssigkeit aus dem Ventilköper 1 heraustransportiert wird, auf einen bestimmten Wert anzupassen, kann es erforderlich sein, den Durchmesser der Mikrokanäle 5, mithin den Abstand der Begrenzungselemente 6 zueinander entsprechend abzustimmen.

Die in den Figuren 1 bis 3 gezeigte Ausführungsform kann beispielsweise einen Innenraumdurchmesser senkrecht zu den Innenseiten 8 der Seitenwände zwischen ungefähr 1 µm und 500 µm aufweisen. Vorzugsweise beträgt dieser Durchmesser zwischen 10 µm und 100 µm.

Der Durchmesser der Mikrokanäle 5, mithin der freie Abstand oder das lichte Maß zwischen den Begrenzungselementen 6, kann zwischen 0,5 und 50 µm liegen. Vorzugsweise beträgt der freie Abstand oder das lichte Maß zwischen den Begrenzungselementen 6 zwischen 3 und 15 µm.

Das erfindungsgemäße Ventil hat dadurch den Vorteil, dass die erforderlichen Strukturen mit Hilfe gängiger Mikrostrukturierungsverfahren hergestellt werden können, beispielsweise mit Hilfe von Mikrospritzguss-oder Siliziumätzverfahren.

### Bezugszeichenliste

- 1: Ventilkörper
- 2: Innenraum
- 3: Flüssigkeitseinlass
- 4: Flüssigkeitsauslass
- 5: Mikrokanal
- 6: Begrenzungselement
- 7: Seitenwand
- 8: Innenseite
- x: Verbindungsrichtung zwischen dem Flüssigkeitseinlass und dem Flüssigkeitsauslas

## Patentansprüche

1. Vorrichtung zur Verabreichung eines flüssigen Medikaments, mit einem Reservoir, in dem ein Medikament vorgehalten oder vorhaltbar ist, und mit einer Pumpe, die eine Pumpenkammer aufweist, die über ein nur in Richtung vom Reservoir in die Pumpenkammer durchlässiges Einwegventil mit dem Reservoir fluidisch verbunden ist, und die über ein Ventil mit einem Medikamentenauslass fluidisch verbunden ist, wobei das Ventil einen Ventilkörper (1) aufweist, der einen Innenraum (2) zur Aufnahme einer Flüssigkeit (20) aufweist, wobei der Ventilkörper (1) einen Flüssigkeitseinlass (3) und einen gegenüber liegenden Flüssigkeitsauslass (4) aufweist, die beide in den Innenraum (2) münden, wobei in dem Innenraum (2) eine Vielzahl Mikrokanäle (5) angeordnet ist, die sich in Verbindungsrichtung (x) zwischen dem Flüssigkeitseinlass (3) und dem Flüssigkeitsauslass (4) erstrecken,
bei dem die Mikrokanäle von einem Gitter aus parallelen, stabförmigen Begrenzungselementen (6), oder von mehreren parallelen, zueinander versetzt angeordneten Lagen eines Gitters aus parallelen, stabförmigen Begrenzungselementen (6) gebildet sind,
wobei die Gesamtheit der parallelen, stabförmigen Begrenzungselemente (6) im Ventilkörper (1) im Innenraum (2) angeordnet ist,
**dadurch gekennzeichnet, dass** der Innenraum (2), der Flüssigkeitseinlass (3) und der Flüssigkeitsauslass (4) dieselbe Querschnittsfläche senkrecht zur Verbindungsrichtung (x) aufweisen.

2. Vorrichtung nach Anspruch 1, wobei das Ventil ein Auslassventil ist.

3. Vorrichtung nach Anspruch 1 oder 2, wobei der Innenraum Seitenwände mit Innenseiten (8) mit einem Innenraumdurchmesser senkrecht zu den Innenseiten zwischen 1 µm bis 500 µm, vorzugsweise zwischen 10 µm und 100 µm aufweist.

4. Vorrichtung nach einem der Ansprüche 1 bis 3, bei der die Mikrokanäle (5) einen Durchmesser zwischen 1 µm und 200 µm und bevorzugt zwischen 5 µm und 20 µm aufweisen.

5. Vorrichtung nach einem der Ansprüche 1 bis 4, bei dem der Durchmesser der Mikrokanäle (5) zwischen 0,5 µm und 50 µm, vorzugsweise zwischen 3 µm und 15 µm liegt.

6. Vorrichtung einem der Ansprüche 1 bis 5, bei der sich die Begrenzungselemente (6) senkrecht zur Verbindungsrichtung (x) zwischen dem Flüssigkeitseinlass (3) und dem Flüssigkeitsauslass (4) erstrecken.

7. Vorrichtung nach einem der Ansprüche 1 bis 6, bei der eine Länge der Begrenzungselemente (6) zwischen 20% und 80% des Durchmessers des Innenraums (2) senkrecht zur Verbindungsrichtung zwischen Flüssigkeitseinlass (3) und Flüssigkeitsauslass (4) beträgt, wobei sich die Begrenzungselemente (6) ausgehend von einer Innenseite (8) der den Innenraum (2) des Ventilkörpers (1) begrenzenden Seitenwand (7) in Richtung einer gegenüberliegenden Seitenwand (7) erstrecken, ohne diese zu erreichen, so dass ein Abstand zwischen den Begrenzungselementen (6) und der jeweils gegenüberliegenden Seitenwand (7) ausgebildet ist.

8. Vorrichtung nach einem der Ansprüche 1 bis 7, bei der die Begrenzungselemente (6) einen runden, insbesondere kreisrunden, oder einen polygonalen Querschnitt aufweisen.

9. Vorrichtung nach einem der vorangegangenen Ansprüche, bei der der Ventilkörper (1) parallele Seitenwände (7) aufweist, deren Innenseiten (8) den Innenraum (2) abgrenzen, wobei die Seitenwände an gegenüberliegenden Enden in den Flüssigkeitseinlass (3) bzw. den Flüssigkeitsauslass (4) münden.

10. Vorrichtung nach Anspruch 9 bei der der Ventilkörper (1) über seine gesamte Länge zwischen dem Flüssigkeitseinlass (3) und dem Flüssigkeitsauslass (4) einen konstanten Querschnitt aufweist.

11. Vorrichtung nach einem der vorangegangenen Ansprüche, bei der der Ventilkörper (1) einen runden, insbesondere kreisrunden, oder einen polygonalen Querschnitt aufweist.

12. Vorrichtung nach einem der vorangegangenen Ansprüche, bei der zur Erhöhung der Adhäsion zwischen den Begrenzungselementen (6) und einer Flüssigkeit die Oberfläche der Begrenzungselemente (6) eine funktionale Beschichtung, vorzugsweise eine hydrophile Beschichtung, aufweist.

13. Vorrichtung nach einem der vorangegangenen Ansprüche, bei der das Querschnittsflächenverhältnis zwischen den Mikrokanälen (5) und dem Innenraum (2) zwischen 1:5 und 1:1000, bevorzugt zwischen 1:50 und 1:100 beträgt.

14. Vorrichtung nach einem der vorangegangenen Ansprüche, bei der die erforderlichen Strukturen des Ventils mit Hilfe von Mikrospritzguss- oder Siliziumätzverfahren hergestellt werden.

## Claims

1. Device for administering a liquid medicament, having a reservoir in which a medicament is held or can be held, and having a pump with a pump chamber which is fluidically connected to the reservoir via a one-way valve allowing medium to pass through only in the direction from the reservoir into the pump chamber and which is fluidically connected to a medicament outlet via a valve, wherein the valve has a valve body (1) which has an inner space (2) for receiving a liquid (20), wherein the valve body (1) has a liquid inlet (3) and, opposite the latter, a liquid outlet (4), which both open into the inner space (2), wherein a multiplicity of microchannels (5) are arranged in the inner space (2), said microchannels (5) extending in connection direction (x) between the liquid inlet (3) and the liquid outlet (4),
wherein the microchannels are formed from a grid of parallel, rod-shaped boundary elements (6), or from a plurality of parallel, mutually offset layers of a grid of parallel, rod-shaped boundary elements (6),
wherein all of the parallel, rod-shaped boundary elements (6) in the valve body (1) are arranged in the inner space (2),
**characterized in that** the inner space (2), the liquid inlet (3) and the liquid outlet (4) have the same cross-sectional area perpendicular to the connection direction (x).

2. Device according to Claim 1, wherein the valve is an outlet valve.

3. Device according to Claim 1 or 2, wherein the inner space has side walls with inner faces (8), with an inner-space diameter perpendicular to the inner faces of between 1 µm and 500 µm, preferably of between 10 µm and 100 µm.

4. Device according to any one of Claims 1 to 3, wherein the microchannels (5) have a diameter of between 1 µm and 200 µm and preferably of between 5 µm and 20 µm.

5. Device according to any one of Claims 1 to 4, wherein the diameter of the microchannels (5) is between 0.5 µm and 50 µm, preferably between 3 µm and 15 µm.

6. Device according to any one of Claims 1 to 5, wherein the boundary elements (6) extend perpendicular to the connection direction (x) between the liquid inlet (3) and the liquid outlet (4).

7. Device according to any one of Claims 1 to 6, wherein a length of the boundary elements (6) measures between 20% and 80% of the diameter of the inner space (2) perpendicular to the connection direction between liquid inlet (3) and liquid outlet (4), wherein the boundary elements (6), starting from an inner face (8) of the side wall (7) bordering the inner space (2) of the valve body (1), extend in the direction of an opposite side wall (7), without reaching the latter, such that a spacing between the boundary elements (6) and the respectively opposite side wall (7) is formed.

8. Device according to any one of Claims 1 to 7, wherein the boundary elements (6) have a round, in particular circular, cross section or a polygonal cross section.

9. Device according to any one of the preceding claims, wherein the valve body (1) has parallel side walls (7) whose inner faces (8) delimit the inner space (2), wherein the side walls open at opposite ends into the liquid inlet (3) or the liquid outlet (4).

10. Device according to Claim 9, wherein the valve body (1) has a constant cross section along its entire length between the liquid inlet (3) and the liquid outlet (4).

11. Device according to any one of the preceding claims, wherein the valve body (1) has a round, in particular circular, cross section or a polygonal cross section.

12. Device according to any one of the preceding claims, wherein, in order to increase the adhesion between the boundary elements (6) and a liquid, the surface of the boundary elements (6) has a functional coating, preferably a hydrophilic coating.

13. Device according to any one of the preceding claims, wherein the cross-sectional area ratio between the microchannels (5) and the inner space (2) is between 1:5 and 1:1000, preferably between 1:50 and 1:100.

14. Device according to any one of the preceding claims, wherein the required structures of the valve are produced by micro-injection moulding or silicon etching processes.

## Revendications

1. Dispositif d'administration d'un médicament liquide, ledit dispositif comprenant un réservoir, dans lequel est ou peut être stocké un médicament, et une pompe qui comporte une chambre de pompe qui est reliée fluidiquement au réservoir par le biais d'une valve unidirectionnelle qui est passante uniquement dans le sens allant du réservoir à la chambre de pompe et qui est reliée fluidiquement à une sortie de médicament par le biais d'une valve, la valve comportant un corps de valve (1) qui comporte un espace intérieur (2) destiné à recevoir un liquide (20), le corps de valve (1) comportant une entrée de liquide (3) et une sortie de liquide opposée (4) qui débouchent toutes les deux dans l'espace intérieur (2), un grand nombre de micro-canaux (5) étant disposés dans l'espace intérieur (2) et s'étendant dans la direction de liaison (x) entre l'entrée de liquide (3) et la sortie de liquide (4),
les micro-canaux étant formés par une grille d'éléments de délimitation parallèles (6) en forme de tige ou par plusieurs couches parallèles décalées entre elles d'une grille d'éléments de délimitation parallèles (6) en forme de tige,
la totalité des éléments de délimitation parallèles (6) en forme de tige dans le corps de valve (1) étant disposée dans l'espace intérieur (2),
**caractérisé en ce que**
l'espace intérieur (2), l'entrée de liquide (3) et la sortie de liquide (4) ont la même aire en coupe transversale perpendiculairement à la direction de liaison (x).

2. Dispositif selon la revendication 1, la valve étant une valve de sortie.

3. Dispositif selon la revendication 1 ou 2, l'espace intérieur comportant des parois latérales qui est pourvues de côtés intérieurs (8) et dont le diamètre perpendiculairement aux côtés intérieurs est compris entre 1 µm et 500 µm, de préférence entre 10 µm et 100 µm.

4. Dispositif selon l'une des revendications 1 à 3, dans lequel les micro-canaux (5) ont un diamètre compris entre 1 µm et 200µm et de préférence entre 5 µm et 20 µm.

5. Dispositif selon l'une des revendications 1 à 4, dans lequel le diamètre des micro-canaux (5) est compris entre 0,5 µm et 50 µm, de préférence entre 3 µm et 15 µm.

6. Dispositif selon l'une des revendications 1 à 5, dans lequel les éléments de délimitation (6) s'étendent perpendiculairement à la direction de liaison (x) entre l'entrée de liquide (3) et la sortie de liquide (4).

7. Dispositif selon l'une des revendications 1 à 6, dans lequel une longueur des éléments de délimitation (6) est comprise entre 20 % et 80 % du diamètre de l'espace intérieur (2) perpendiculairement à la direction de liaison entre l'entrée de liquide (3) et la sortie de liquide (4), les éléments de délimitation (6) s'étendant depuis un côté intérieur (8) de la paroi latérale (7) qui délimite l'espace intérieur (2) du corps de valve (1) en direction d'une paroi latérale opposée (7) sans atteindre celle-ci de sorte qu'une distance est ménagée entre les éléments de délimitation (6) et la paroi latérale opposée (7) respective.

8. Dispositif selon l'une des revendications 1 à 7, dans lequel les éléments de délimitation (6) présentent une section transversale ronde, notamment circulaire, ou polygonale.

9. Dispositif selon l'une des revendications précédentes, dans lequel le corps de valve (1) comporte des parois latérales parallèles (7) dont les côtés intérieurs (8) délimitent l'espace intérieur (2), les parois latérales débouchant, au niveau des extrémités opposées, dans l'entrée de liquide (3) ou la sortie de liquide (4).

10. Dispositif selon la revendication 9, dans lequel le corps de valve (1) présente une section transversale constante sur toute sa longueur entre l'entrée de liquide (3) et la sortie de liquide (4).

11. Dispositif selon l'une des revendications précédentes, dans lequel le corps de valve (1) présente une section transversale ronde, notamment circulaire, ou polygonale.

12. Dispositif selon l'une des revendications précédentes, dans lequel la surface des éléments de délimitation (6) comporte un revêtement fonctionnel, de préférence un revêtement hydrophile, afin d'augmenter l'adhérence entre les éléments de délimitation (6) et un liquide.

13. Dispositif selon l'une des revendications précédentes, dans lequel le rapport des aires en coupe transversale entre les micro-canaux (5) et l'espace intérieur (2) est compris entre 1:5 et 1:1000, de préférence entre 1:50 et 1:100.

14. Dispositif selon l'une des revendications précédentes, dans lequel les structures nécessaires de la valve sont fabriquées au moyen de procédés de micro-moulage par injection ou de gravure du silicium.
